# EUROPEAN PATENT APPLICATION

(11) **EP 3 061 820 A1**
(43) Date of publication of application: **31.08.2016**
(21) Application number: 15156650.2
(22) Date of filing: 26.02.2015
(51) Int. Cl.: C12N 15/09

(54) **Rapid depletion and reversible accumulation of proteins in vivo**

(71) Applicant: Leibniz-Institut für Pflanzenbiochemie (IPB), 06120 Halle (Saale) (DE); CENTRE NATIONAL DE LA RECHERCHE SCIENTIFIQUE -CNRS-, 75016 Paris (FR)
(72) Inventor: Dissmeyer, Nico. Dr., 06114 Halle (Saale) (DE); Schnittger, Arp, 22609 Hamburg (DE)
(74) Representative: Schrell, Andreas

(57) **Abstract**

The present invention relates to a process for producing a temperature-sensitive conditionally mutant multicellular organism using a low-temperature N-terminal degradation cassette or a vector comprising it to transfect or transform at least one cell or part of a multicellular organism, thereby obtaining the temperature-sensitive conditional mutant of the multicellular organism or part thereof. Further, the present invention relates to a low-temperature N-terminal degradation cassette comprising nucleotide sequences encoding at least a temperature-sensitive DHFR protein, a destabilizing amino acid, a ubiquitin moiety, as well as a protein of interest.

## Description

The present invention relates to a process for producing a temperature-sensitive conditionally mutant multicellular organism using a low-temperature N-terminal degradation cassette or a vector comprising it to transfect or transform at least one cell or part of a multicellular organism, thereby obtaining the temperature-sensitive conditional mutant of the multicellular organism or part thereof. Further, the present invention relates to a low-temperature N-terminal degradation cassette comprising nucleotide sequences encoding at least a temperature-sensitive DHFR protein, a destabilizing amino acid, a ubiquitin moiety, as well as a protein of interest.

Temperature-sensitive mutants are a powerful tool with which to study gene function in vivo. Such temperature-sensitive mutants are ones in which there is a marked drop in the level or activity of the gene product when the gene is expressed above a certain temperature, the so-called restrictive temperature. Below this temperature, at so-called permissive temperatures, the activity or phenotype of the mutant is similar to that of the wildtype. Thus, temperature-sensitive mutants provide an extremely powerful tool for studying protein function and assembly in vivo, because these mutants provide a reversible mechanism to lower the level of a specific gene product at any stage in the growth of the organism simply by changing the temperature of growth.

Thus, phenotypes can be generated on demand by controlling activation and accumulation of proteins of interest which are invaluable tools to analyze and engineer biological processes. While temperature-sensitive alleles are frequently used as conditional mutants in microorganisms, they are difficult to identify in multicellular species.

Although there are other inducible systems for gene expression, temperature-sensitive mutants have several unique advantages, including fast temporal response, high reversibility, and the applicability to any tissue type or developmental stage of an organism. To date, temperature-sensitive mutants of a protein of interest are generated by random mutagenesis, typically with a chemical mutagen, often followed by laborious screening of large numbers of progeny. Thus, this procedure is usually limited to fast-growing monocellular organisms where mutated populations can be analyzed simultaneously at the restrictive and the permissive temperatures.

In an alternative approach, heat-sensitive mutants are generated using a temperature-inducible N-degron, where a protein of interest is fused to a portable N-terminal degron. In particular in yeast, it was shown that temperature-sensitive mutants can be generated by fusing a protein of interest to a temperature-sensitive degron. Temperature-sensitive degrons represent specific degradation signals and take advantage of the N-end rule pathway of targeted protein degradation (NERD). The fundamental principles of the N-end rule and the proteolytic pathway that implements it are well-established in the literature (see, e.g., Bachmair et al., Science 234:179 (1986); Varshavsky, Cell 69:725 (1992)). The classical yeast N-degron is based on a temperature-sensitive mutant of mouse dihydrofolate reductase (DHFR) containing a single amino acid substitution at position 67 (US 5,705,387). This temperature-sensitive DHFR mutant is fused to an N-terminal destabilizing amino acid according to the N-end rule degradation pathway which dramatically decreases the in vivo half-life of a protein. This degron construct containing the mutant DHFR protein is heat-labile at temperatures above 37°C, leading to exposure of an internal lysine residue, the site of poly-ubiquitination. A protein of interest fused to this degron is targeted via poly-ubiquitination for proteasome dependent proteolysis upon shifting to a restrictive temperature.

Thus, by using this N-degron technique, proteins of interest can be expressed conditionally to establish phenotypes on demand by controlling activation and inactivation of these proteins via temperature shift from permissive to restrictive temperatures. This method can thus be used as a rapid on/off switch for protein expression based on temperature, e.g. for supplementing a central gene product in its mutated background. However, to date this system has only been used at the single-cell level in yeast and cell culture where it requires restrictive temperatures of 37 or 42° C. These high temperatures are beyond the physiological range of many multicellular organisms, e.g. plants. Accordingly, up to now it is not possible to provide multicellular organisms, in particular plants, which as a whole allow temperature-dependent conditional protein expression or wherein specific reaction compartments, in particular specific cells or specialized tissues thereof, are susceptible to a temperature-dependent switching of protein expression. Up to now, there are no multicellular organisms, in particular plants, available which are capable of conditional, i.e. temperature dependent, development of reaction compartments, that means specific cells or tissues whose presence and/or development can be controlled by switching protein expression on or off in a temperature-dependent way, whereby the protein expression controls and allows the development of said reaction compartment.

The technical problem underlying the present invention is thus to overcome the aforementioned disadvantages and in particular to provide methods, multicellular organisms and means to produce temperature-sensitive conditional mutants of multicellular organisms with restrictive temperatures tolerable by multicellular organisms like plants or animals. One particular technical problem underlying the present invention is further to provide multicellular organisms, in particular plants, which comprise conditional reaction compartments which can be generated via temperature-dependent protein expression and used for example as conditional bioreactors. This technical problem is solved by the subject-matter of the independent claims, in particular by a process for producing a temperature-sensitive conditional mutant of a multicellular organism comprising the steps of:
a) providing a low-temperature N-terminal degradation cassette or a vector comprising it and at least one cell or part of a multicellular organism,
b) transfecting or transforming the at least one cell or part of the multicellular organism with the low-temperature N-terminal degradation cassette or vector, and
c) obtaining a temperature-sensitive conditional mutant of the multicellular organism or a part thereof.

The present invention therefore uses as starting material, a low-temperature N-terminal degradation cassette or a vector comprising a low-temperature N-terminal degradation cassette and at least one cell or part of a multicellular organism. Said at least one cell or part of the multicellular organism, such as suitable animal cells or a plant part, in particular root, or a callus, is preferably a wildtype, in particular non-mutated, cell or part of a multicellular organism. By transfecting or transforming the at least one cell or part of the multicellular organism with the low-temperature N-terminal degradation cassette or the vector containing it, a temperature-sensitive conditional mutant of said multicellular organism or part thereof is obtained. Preferably, the whole multicellular organism can be generated from the at least one transfected or transformed cell or the transfected or transformed part of the multicellular organism.

Advantageously, by using the present low-temperature N-terminal degradation cassette it is possible to create temperature-sensitive conditional mutants of multicellular organisms because the restrictive temperatures are lower and thus tolerable by multicellular organisms. The present invention therefore presents a versatile and transferable genetically stable system based on a low-temperature-controlled N-terminal degradation signal (N-degron) that allows reversible and switch-like tuning of protein levels under physiological conditions in vivo. It is thereby possible to trigger developmental effects and to generate phenotypes on demand.

In a preferred embodiment, the low-temperature N-terminal degradation cassette used in the present process comprises:
i) a nucleotide sequence encoding a temperature-sensitive DHFR protein, which is a DHFR protein, in which at least one amino acid selected from the group consisting of the residues corresponding to threonine 39, valine 51, isoleucine 52, methionine 53, valine 75, valine 113, tryptophan 114, isoleucine 115, leucine 134, phenylalanine 135, valine 136, isoleucine 139 and glutamic acid 173 of the wildtype mouse DHFR protein according to SEQ ID No. 1 is substituted,
ii) a codon for a destabilizing amino acid selected from the group consisting of arginine, lysine, histidine, phenylalanine, tyrosine, tryptophan, leucine, isoleucine, methionine, aspartic acid, glutamic acid, asparagine, glutamine and cysteine which codon is located 5' of the nucleotide sequence encoding the temperature-sensitive DHFR protein identified in i), and
iii) a nucleotide sequence encoding a ubiquitin moiety which is located 5' of the codon for the destabilizing amino acid identified in ii).

Thus, advantageously, the present low-temperature degradation cassette comprises a temperature-sensitive DHFR protein as identified in i) above, in which at least one of the above identified amino acid residues is substituted. Surprisingly, the inventors found that a DHFR protein comprising at least one of those amino acid substitutions is temperature-sensitive and becomes unstable at much lower temperatures compared to the prior art. Thus, an N-terminal degradation cassette comprising the present temperature-sensitive DHFR protein is degraded at lower temperatures, that means the restrictive temperature is lower, than that of known temperature-sensitive N-degrons. It is thus possible to enable conditional, temperature-dependent protein expression in multicellular organisms under physiological conditions. As with the classical yeast N-degron, degradation of the N-terminal degradation cassette is effected at restrictive temperatures via the N-end rule pathway and the proteasome. Thus, transcript levels of the low-temperature degradation cassette are unaffected by temperature shifts but the resulting protein is rapidly degraded by the proteasome at restrictive temperatures, whereas the protein is stable at permissive temperatures.

In particular, the permissive temperature at which the present degradation cassette is expressed and the protein is stable in the transfected or transformed cells is below 24 °C, whereas the restrictive temperature at which the degradation cassette is unstable and the protein is degraded is as low as 24 °C and above.

As specified in ii) above, the present degradation cassette further comprises a codon for a destabilizing amino acid which is located 5' of the nucleotide sequence encoding the temperature-sensitive DHFR protein. In particular, the destabilizing amino acid is an amino acid according to the N-end rule pathway, preferably type I primary destabilizing amino acids such as arginine, lysine or histidine; type II primary destabilizing amino acids such as phenylalanine, tyrosine, tryptophan, leucine, isoleucine or methionine; secondary destabilizing amino acids such as aspartic acid or glutamic acid; or tertiary destabilizing amino acids such as asparagine, glutamine or cysteine. Preferably, the destabilizing amino acid is selected from the group consisting of arginine, phenylalanine and leucine. Particularly preferred, the destabilizing amino acid is phenylalanine.

Furthermore, as specified in iii) above, the present degradation cassette comprises a nucleotide sequence encoding a ubiquitin moiety which is located 5' of the codon for the destabilizing amino acid. The ubiquitin moiety is removed cotranslationally from the expressed fusion protein by deubiquitinating enzymes and the destabilizing amino acid is exposed. Thus, by using an N-terminal ubiquitin moiety the destabilizing amino acid of choice can be produced at the N-terminus of the temperature-sensitive DHFR. Concomitantly with deubiquitinating, previously internal lysine residues of the temperature-sensitive DHFR protein become surface exposed and act as poly-ubiquitin acceptors at restrictive temperatures, which in turn targets the whole protein for proteasome-dependent degradation. Particularly preferred, the nucleotide sequence encoding the ubiquitin moiety is codon optimized for the specific multicellular organism used. Thus, e.g. the nucleotide sequence encoding the ubiquitin moiety is a plant optimized ubiquitin moiety nucleotide sequence with an optimized sequence according to the codon usage of the respective species.

As discussed above, the present low-temperature degradation cassette comprises as component i) a nucleotide sequence encoding a temperature-sensitive DHFR protein, which comprises at least one of the above identified amino acid substitutions. Said nucleotide sequence encoding the DHFR protein can be derived from any suitable species, for instance animals, such as mammals, in particular rat, human or mouse, plants or protozoa, provided that the protein has dihydrofolate reductase activity and comprises at least the amino acid residues corresponding to the specifically identified amino acid residues in feature i) to be substituted. Said specifically identified amino acid residues are those from which at least one, preferably two, three or more, are substituted by another amino acid. The position of said specifically identified amino acid residues which are to be substituted in the DHFR protein is given with respect to a reference DHFR protein, namely the wildtype mouse DHFR protein according to SEQ ID No. 1. Thus, the mouse DHFR amino acid sequence SEQ ID No. 1 referred to in feature i) serves only as reference for the position of the amino acids to be substituted and does not limit the invention to the particular DHFR sequence. Due to the high level of conservation one skilled in the art is able to find the nucleotide and amino acid sequence corresponding to the mouse DHFR sequence in other species.

In a preferred embodiment, the degradation cassette comprises a nucleotide sequence encoding the wildtype mouse DHFR protein comprising at least one of the above identified amino acid substitutions. Such a preferred temperature-sensitive DHFR protein is represented by SEQ ID No. 2, wherein all putatively substituted amino acid residues, which are threonine 39, valine 51, isoleucine 52, methionine 53, valine 75, valine 113, tryptophan 114, isoleucine 115, leucine 134, phenylalanine 135, valine 136, isoleucine 139 and glutamic acid 173, are replaced by Xaa.

In a preferred embodiment, the temperature-sensitive DHFR protein comprises at least one amino acid substitution of one of the amino acids residues specified above, wherein in case any of the valine residues 51, 75, 113 and 136 is substituted, then it is substituted with an amino acid selected from the group consisting of glycine, asparagine and threonine; in case any of the residues isoleucine 52, methionine 53, tryptophan 114, isoleucine 115, leucine 134, phenylalanine 135 and isoleucine 139 is substituted, then it is substituted with an amino acid selected from the group consisting of glycine, glutamic acid and asparagine; in case the threonine residue 39 is substituted, then it is substituted with alanine and in case the glutamic acid residue 173 is substituted, then it is substituted with aspartic acid. Particularly preferred, at least one of the amino acids threonine 39 and glutamic acid 173 are substituted. Most preferred the amino acid glutamic acid 173 is substituted.

In a preferred embodiment of the present invention, the threonine residue 39 of the DHFR protein is substituted with alanine and/or the glutamic acid residue 173 is substituted with aspartic acid, preferably both amino acids are substituted as represented by SEQ ID No. 3. Most preferred only the amino acid glutamic acid 173 is substituted with aspartic acid.

In a preferred embodiment of the present invention, the degradation cassette used in the present process further comprises:
iv) a nucleotide sequence encoding a protein of interest A, B or a combination thereof which is located 3' of the nucleotide sequence encoding the temperature-sensitive DHFR protein identified in i).

Thus, the present degradation cassette preferably also comprises a nucleotide sequence encoding a protein of interest A, B or a combination thereof. This protein of interest A or B is thus expressed and accumulated in a cell which has been transfected or transformed with the present degradation cassette at a permissive lower temperature, whereas it is degraded at a restrictive higher temperature because it is fused to the temperature-sensitive DHFR protein of the present invention.

Thus, in a preferred embodiment, the protein of interest A, B or the combination thereof is degradable upon shifting the temperature-sensitive conditional mutant of the multicellular organism from a permissive lower temperature to a restrictive higher temperature for a period of time sufficient to facilitate degradation of the protein of interest A, B or the combination thereof in the cells of the mutant multicellular organism.

In a preferred embodiment, the degradation cassette thus comprises from 5' to 3' nucleotide sequences encoding: the ubiquitin moiety (iii), the destabilizing amino acid (ii), the temperature-sensitive DHFR protein (i) and preferably the protein of interest A and/or B (iv). At permissive temperatures, this degradation cassette is expressed in the transfected or transformed cells of the multicellular organism and the protein of interest A and/or B is expressed and accumulated in the cell as a fusion protein containing the above-mentioned elements. Preferably, being expressed as such a fusion protein does not hinder the function of the protein of interest A and/or B. However, at restrictive higher temperatures, the degradation cassette is still expressed but the resulting fusion protein is rapidly degraded via poly-ubiquitination by the proteasome.

In a preferred embodiment of the present invention, the multicellular organism, part or at least one cell thereof provided in step a) of the present process lacks a functional endogenous gene encoding the protein of interest A and/or B. Thus, the protein of interest A or B which is provided via the transfected or transformed degradation cassette supplements the protein A and/or B, thus allowing temperature-dependent expression of the protein of interest A and/or B.

In a preferred embodiment of the present invention, the protein of interest A is a protein to be produced in a transfected or transformed cell in a temperature-dependent manner and which is of commercial or research interest. In a preferred embodiment, the protein of interest A is a protein improving the nutritional value of a plant, conveying tolerance against biotic or abiotic stresses, an enzyme, a pharmaceutical or nutraceutical protein, a medicinal enzyme or a protein that can be used for diagnostic, industrial or technical purposes. Preferred examples for a protein of interest A are storage proteins, amidases, peroxidases, viral core antigens, antibodies, vaccines, hormones and growth factors or proteinaceous toxins such as lectins. In case the protein of interest A is an enzyme, as e.g. TEV, it can preferably also be used for intracellular cleavage of a substrate which is either naturally occurring in the cell or has been transfected or transformed into the cell as well, e.g. as a protein of interest C (see below). Preferred proteins of interest A are green fluorescent protein (GFP), β-glucuronidase (GUS), tobacco etch virus protease (TEV), phosphinothricin-N-acetyltransferase (PAT) which is able to provide herbicide resistance and cyclin-dependent kinase (CDKA;1) which is able to increase cell division competency.

In a preferred embodiment of the present invention, the protein of interest B is able to control the development of a reaction compartment of the multicellular organism. In this way, the multicellular organism obtained in step c) of the present process is able to develop the reaction compartment temperature-dependently. In particular, the protein of interest B is a protein which controls the development of a reaction compartment of the multicellular organism either positively or negatively. Thus, in a preferred embodiment the reaction compartment is developed by the mutant multicellular organism at permissive temperatures, which means that the protein of interest B is accumulated in the cells at permissive temperatures and the protein of interest B affects the development of the reaction compartment positively, that means the expressed protein of interest B allows the development of the reaction compartment. In this case, the multicellular organism preferably lacks a functional gene encoding the endogenous protein of interest B. In another preferred embodiment, the protein of interest affects the development of the reaction compartment negatively, e.g. in form of a repressor. In this case, the reaction compartment can only be developed by the mutant multicellular organism at restrictive temperatures when the protein of interest B is degraded. Thus, preferably, the multicellular organism is allowed to grow under permissive or restrictive temperatures depending on the nature of the protein of interest B for a period of time sufficient for development of the reaction compartment.

In the context of the present invention, the reaction compartment is a specific tissue, cell type or organ of the multicellular organism. Particularly preferred, the reaction compartment(s) are trichomes (leaf hairs), floral meristem cells, stomata (epidermal pores) or specific cell types of the root, seed or entire silique, such as root hairs, seed coat or endosperm. Thus, preferably, the protein of interest B is a protein able to control the development of the above-mentioned tissues, cell types or organs, in particular of trichomes or floral meristem cells of a suitable plant. For example, protein of interest B preferably is TTG1 (TRANSPARENT TESTA GLABRA 1), other members of the GLABRA (GL) family or related proteins involved in trichome development, or CO (CONSTANS) and related proteins involved in the development of floral meristem cells in *Arabidopsis thaliana,* wherein the plant is either a mutant or wildtype with respect to protein of interest B. Other examples are members of the APETALA (AP) family, LEAFY (LFY), AGAMOUS (AGA) or similar candidates involved in flower development, endosperm or fruit specific factors such as hordein family members and similar target proteins.

In general, to be a suitable candidate for protein of interest A or B the respective protein must on the one hand - at least transiently - capable to localize or be enabled to localize to cellular compartments where the N-end rule pathway is active such as the cytosol and the nucleus and on the other hand cause developmental phenotypes in loss-of-function or gain-of-function.

In a preferred embodiment of the present process, the at least one cell or part of the multicellular organism is transfected or transformed in step b) of the present process with a degradation cassette comprising a nucleotide sequence encoding at least the protein of interest B and simultaneously or in a further step d) with a vector comprising a nucleotide sequence encoding at least a protein of interest C which nucleotide sequence is under the control of a promotor specific for the reaction compartment, which development is controlled by the protein of interest B, and wherein in step c) or a further step e) a multicellular organism is obtained which is able to develop the reaction compartment and to express the protein of interest C in the reaction compartment temperature-dependently. Preferably, in case the reaction compartments are *Arabidopsis thaliana* trichomes promotors like ProTRIPTYCHON, ProGLABRA2 or ProCAPRICE can be used. Further, in case the reaction compartment is meristematic tissue of *Arabidopsis thaliana* promotors like ProCDKA;1 or ProWUSCHEL can be used.

Thus, advantageously, it is possible to create a conditional reaction compartment which is developed by the mutated multicellular organism at permissive or restrictive temperatures depending on the protein of interest B. Furthermore, by transfecting or transforming the multicellular organism also with a nucleotide sequence encoding a protein of interest C which itself is under the control of a promotor specific for the reaction compartment developed depending on the protein of interest B, the protein of interest C is only expressed in the cells of the reaction compartment although every cell of the mutant multicellular organism might comprise the vector encoding the protein of interest C. Preferably, the protein of interest C is an enzyme, an enzymatic cascade or part of an enzymatic cascade or at least one regulatory element thereof, such as for example one or more transcription factors, so that the protein of interest C is able to directly or indirectly generate a molecule, in particular a small molecule, or for instance a metabolite, of interest. Preferably, the molecule or metabolite generated is an anthocyanin. In this case, protein of interest C preferably is at least one transcription factor, more preferably two transcription factors, in particular DELILA and ROSEA1 in *Arabidopsis thaliana*. Successful generation of anthocyanin in the reaction compartment, preferably trichomes, is preferably indicated by violet coloring. Furthermore, the molecule or metabolite generated can be a protein. Thus, advantageously, the conditional reaction compartment(s) of the mutated multicellular organism can be used as bioreactors for the production of molecules, metabolites or proteins of interest, which can be regulated temperature-dependently. This is especially advantageous in case the produced molecules or proteins are toxic for the whole multicellular organism but not for certain parts thereof or are only toxic during specific phases of development of the multicellular organism. Preferred toxic proteins of interest C are Barnase, a highly toxic bacterial ribonuclease from *Bacillus amyloliquifaciens,* and *Diphtheria* toxin A, a potent inhibitor of protein biosynthesis. Toxicity of these proteins is lost at restrictive temperatures because the protein is degraded, whereas at permissive temperatures cells of the reaction compartment get specifically ablated.

In a preferred embodiment, the protein of interest C is a developmental regulator such as a transcription factor or a repressor. Furthermore, the protein of interest C can preferably be a member of one of the six main functional enzyme families comprising oxidoreductases (EC.1), transferases (EC.2), hydrolases (EC.3), lyases (EC.4), isomerases (EC.5), and ligases (EC.6). In particular, the protein of interest C is a member of one of the 24 sub-family classes of EC.1 (oxidoreductases), a member of one of the 10 sub-family classes of EC.2 (transferases), a member of one of the 13 sub-family classes of EC.3 (hydrolases), a member of one of the 8 sub-family classes of EC.4 (lyases), a member of one of the 6 sub-family classes of EC.5 (isomerases), or a member of one of the 6 sub-family classes of EC.6 (ligases). More preferably, it is a protease, a kinase, a glucosyltransferase, an oxygenase, a hydroxylase, a reductase, a tyrosinase, or a peroxidase.

In a preferred embodiment of the present invention, the molecule, in particular the metabolite, of interest is a phenylpropanoid, a flavonoid, a terpenoid, an alkaloid, a glycoside, a coumarin, more preferably a member of the betalains such as anthocyanin or anthocyanidin, carotinoid or betanin.

In a preferred embodiment of the present invention, the permissive temperature is below 24 °C and the restrictive temperature is at least 24 °C. In particular, the permissive temperature is from 10 °C to 24 °C, preferably from 10 °C to 22 °C, preferably from 12 °C to 20°C, more preferably from 13 °C to 19 °C, preferably from 13 °C to 16 °C, in particular the permissive temperature is 13 °C or 14 °C, whereas the restrictive temperature is from 24 °C to 37 °C, preferably from 24 °C to 35 °C, preferably from 25 °C to 33 °C, preferably from 25 °C to 31 °C, preferably from 25 °C to 30 °C, preferably from 25 °C to 29 °C, more preferably from 25 °C to 28 °C, preferably from 26 °C to 29 °C, in particular the restrictive temperature is 27 °C or 29 °C. Most preferably, the restrictive temperature is determined depending on the specific protein of interest A or B used.

Advantageously, the amount and/or functionality, in particular enzymatic activity, of protein of interest A and/or B is tunable from the maximal amount and/or functionality of said proteins at permissive temperatures over lower amounts at semi-restrictive temperatures up to no or almost no protein amount and/or functionality, in particular enzymatic activity, at restrictive temperatures. For example, semi-restrictive temperatures are from 18 °C to 26 °C, preferably from 19 °C to 25 °C, in particular from 20 to 24 °C.

In a preferred embodiment of the present invention, the multicellular organism is a plant, in particular a flowering plant or an animal, in particular an insect. Most preferably, the multicellular organism is a member of the mustard family (*Brassicaceae*), in particular *Arabidopsis thaliana,* a member of the nightshade family (*Solanaceae*) such as of the genus *Nicotiana* (tobacco), in particular *Nicotiana benthamiana*, or of the genus *Solanum,* in particular *Solanum lycopersicum* (tomato) or *Solanium tuberosum* (potato), or a member of the grass family (*Poaceae*), in particular barley *(Hordeum vulgate*) or maize (*Zea mays*), or *Drosophila melanogaster.*

In a preferred embodiment of the present invention, the degradation cassette used in the present process further comprises:
v) a nucleotide sequence encoding a linker, preferably comprising the amino acids histidine, glycine, serine, glycine and isoleucine, which nucleotide sequence is located 5' of the nucleotide sequence encoding the temperature-sensitive DHFR protein identified in i) and 3' of the codon for the destabilizing amino acid identified in ii).

Preferably, the linker consists of the amino acids histidine, glycine, serine, glycine and isoleucine, preferably the linker consists of five amino acids, namely histidine, glycine, serine, glycine and isoleucine, preferably in this order (HGSGI, SEQ ID no. 30). Advantageously, such a linker enhances molecular flexibility thereby supporting the temperature-sensitivity of the degradation cassette.

In a preferred embodiment of the present invention, the degradation cassette used in the present process further comprises:
vi) a nucleotide sequence encoding a triple hemagglutinin epitope which nucleotide sequence is located 3' of the nucleotide sequence encoding the temperature-sensitive DHFR protein identified in i) and 5' of the nucleotide sequence encoding the protein of interest A, B or the combination thereof identified in iv).

Advantageously, the triple hemagglutinin epitope can be used for immunodetection of the fusion protein expressed by the transfected or transformed cells.

The hemagglutinin epitope is preferably YPYDVPDYA (SEQ ID no. 31), more preferably GSYPYDVPDYA (SEQ ID no. 32).

Thus, in a preferred embodiment, the degradation cassette comprises from 5' to 3' nucleotide sequences encoding: the ubiquitin moiety (iii), the destabilizing amino acid (ii), preferably the linker (v), the temperature-sensitive DHFR protein (i), preferably the triple hemagglutinin epitope (vi) and preferably the protein of interest A and/or B (iv). At permissive temperatures, this degradation cassette is expressed in the transfected or transformed cells of the mutant multicellular organism and the protein of interest A and/or B is expressed and accumulated in the cell as a fusion protein containing at least i), ii) and iii) of the above-mentioned elements, preferably also iv), preferably also v) and most preferably all elements. Preferably, being expressed as such a fusion protein does not hinder the function of the protein of interest A and/or B. However, at restrictive higher temperatures, the degradation cassette is still expressed but the resulting fusion protein is rapidly degraded via poly-ubiquitination by the proteasome.

In a preferred embodiment, the temperature-sensitive conditional mutant of the multicellular organism obtained in step c) or further step e) of the present process contains the low-temperature degradation cassette transiently or stably integrated into its genome.

The present invention also relates to a low-temperature N-terminal degradation cassette as defined above.

In the context of the present invention the term 'at least one amino acid substitution' refers to preferably one amino acid substitution, in particular solely one amino acid substitution. In a further preferred embodiment, the term 'at least one amino acid substitution' refers to two amino acid substitutions, in particular solely two amino acid substitutions. In a further preferred embodiment, the term 'at least one amino acid substitution' refers to three amino acid substitutions, in particular solely three amino acid substitutions. In a further preferred embodiment, the term 'at least one amino acid substitution' refers to four amino acid substitutions, in particular solely four amino acid substitutions. In a further preferred embodiment, the term 'at least one amino acid substitution' refers to five amino acid substitutions, in particular solely five amino acid substitutions.

In a preferred embodiment of the present invention, the at least one amino acid substitution is at least one amino acid substitution, is at least two amino acid substitutions, is at least three amino acid substitutions, is at least four amino acid substitutions or is at least five amino acid substitutions.

In a preferred embodiment of the present invention, the maximum number of amino acid substitutions is two, three, four, five, six, seven, eight, nine, ten, eleven, twelve and, most preferably, thirteen.

In the context of the present invention, a degron is a specific sequence of amino acids in a protein that controls the metabolic stability of a protein and thus makes it short lived in vivo or in vitro. A degron sequence can for instance occur at either the N- or C-terminus and is called N-degron or C-degron, respectively. Further, a degron sequence can also be located within a protein sequence.

In the context of the present invention, specifically N-degrons are concerned, in particular temperature-sensitive N-degrons. Temperature-sensitive N-degrons take advantage of the N-end rule pathway of targeted protein degradation (abbreviated NERD) according to which the N-terminal amino acid of a protein determines its half-life and therefore its likelihood to be degraded (Varshavsky 1997, Genes Cells 2: 13-28). Accordingly, a destabilizing N-terminal residue decreases the in vivo or in vitro half-life of a protein. Thus, an N-degron is an amino-terminal (N-terminal) degradation signal (Johnson, Gonda and Varshavsky, 1990, Nature 346:287-91) which is recognized by and subjects the whole protein to NERD. NERD is part of the ubiquitin proteasome system (UPS) in yeast, animals and plants and of the protease-dependent proteolytic machinery in bacteria. It controls protein stability of cytosolic and nuclear proteins but also for proteins localized in the membrane. Besides the initiating destabilizing amino acid, N-degrons comprise several determinants in order to target a substrate, in particular a protein, to NERD. First, the N-degron must contain a destabilizing N-terminal amino acid that can be recognized by NERD E3 ubiquitin ligases (N-recognins). Second, another crucial factor is a certain flexibility and accessibility of the N-terminal amino acid enabling a proper recognition of the substrate. Third, the N-degron needs to contain at least one internal lysine residue in appropriate distance to the N-terminal amino acid that may serve as poly-ubiquitination site. Thus, in a preferred embodiment, an N-degron as referred to in the present invention, is a degradation signal that contains at least a destabilizing N-terminal amino acid and a temperature-sensitive DHFR, wherein the destabilizing N-terminal amino acid is located at the N-terminus of the DHFR and the DHFR provides the at least one internal lysine residue serving as poly-ubiquitination site.

To be able to engineer the destabilizing amino acid of choice at the N-terminus of the temperature-sensitive DHFR protein the ubiquitin-fusion technique (UFT; Baker et al., 1994, J Biol Chem 269: 25381-6; Varshavsky, 2005, Methods Enzymol 399: 777-99) is used. Thus, the ubiquitin (Ub) moiety serves as an initiating protein which allows engineering of the very N-terminal amino acid of an N-degron which is encoded at the Ub-N-degron junction. Ub is cotranslationally cleaved by deubiquitinating enzymes after its last amino acid (Gly76) and leaves the following residue as neo-N-terminus of the C-terminally fused N-degron. Thereby, UFT leads automatically to the required exposure of the desired destabilizing amino acid at the N-terminus of the N-degron.

In the context of the present invention, a reference to a protein or an N-degron which uses or takes advantage of the N-end rule pathway means that such a protein or N-degron has the property of being recognized by and subjected to NERD in a temperature dependent way.

In the context of the present invention, the term 'protein of interest A or B' means either one protein A or B or refers to more than one protein A or B, for instance, two, three, four, five, six, seven, eight, nine, ten, eleven or more proteins A or B.

In a particularly preferred embodiment, the term 'protein of interest B' may refer to two, three, four, five, six, seven, eight, nine, ten, eleven or more proteins of interest B, which are involved in the development of a reaction compartment.

In a furthermore preferred embodiment, the term 'protein of interest C' may refer to two, three, four, five, six, seven, eight, nine, ten, eleven or more proteins of interest C, which are an enzymatic cascade or a part thereof or regulatory elements thereof responsible for or involved in the regulation of an enzymatic activity or of an enzymatic cascade, for the production of a molecule, preferably metabolite, of interest.

In the context of the present invention 5' is understood to mean upstream from another element within a DNA sequence, whereas 3' is understood to mean downstream from the respective element.

In the context of the present invention, an 'endogenous' gene, allele or protein refers to a non-recombinant sequence of a multicellular organism as the sequence occurs in the respective multicellular organism, in particular wildtype form of the multicellular organism. The term 'mutated' refers to a sequence subjected to a genetic manipulation step, preferably carried out by human interaction.

A nucleotide or amino acid sequence is 'heterologous or exogenous to' an organism if it originates from a foreign species, or, if from the same species, is modified from its original form. 'Recombinant' refers to a human-altered, i.e. transgenic nucleotide or amino acid sequence. A 'transgene' is used as the term is understood in the art and refers to a, preferably heterologous, nucleic acid introduced into a cell by human molecular manipulation of the cell's genome, e.g. by molecular transformation. Thus, a 'transgenic multicellular organism' is a multicellular organism comprising a transgene, i.e. is a genetically-modified multicellular organism. The transgenic multicellular organism can be the initial multicellular organism into which the transgene was introduced as well as progeny thereof whose genome contains the transgene as well.

The term 'nucleotide sequence encoding' or 'coding sequence' refers to a nucleic acid which directs the expression of one or more specific protein(s). The nucleotide sequences include both the DNA strand sequence that is transcribed into RNA and the RNA sequence that is translated into the protein(s). The nucleotide sequences include both the full length nucleic acid sequences as well as non-full length sequences derived from the full length sequences.

The term 'gene' refers to a coding nucleotide sequence and associated regulatory nucleotide sequences.

A 'promoter' is a DNA sequence initiating transcription of an associated DNA sequence, in particular being located upstream (5') from the start of transcription and being involved in recognition and binding of the RNA-polymerase. Depending on the specific promoter region it may also include elements that act as regulators of gene expression such as activators, enhancers, and/or repressors.

The term 'vector' refers to a recombinant DNA construct which may be a plasmid, in particular a Ti-plasmid, a transfer-DNA, a virus, autonomously replicating sequence, an artificial chromosome, such as the bacterial artificial chromosome BAC, phage or other suitable nucleotide sequence. A vector may be linear or circular. A vector may be composed of a single or double stranded DNA or RNA.

The term 'expression' refers to the transcription and/or translation of an endogenous gene or a transgene in a multicellular organism.

'Transfecting' or 'transforming' refers to methods to transfer nucleic acid molecules, in particular DNA, into cells including, but not limited to, biolistic approaches such as particle bombardment, microinjection, permeabilizing the cell membrane with various physical, for instance electroporation, or chemical treatments, for instance polyethylene glycol or PEG, treatments; the fusion of protoplasts or *Agrobacterium tumefaciens* or *rhizogenes* mediated transformation. For the injection and electroporation of DNA in cells there are no specific requirements for the plasmids used.

In the context of the present invention, the term 'comprising' as used herein is understood as to have the meaning of 'including' or 'containing', which means that in addition to the explicitly mentioned element further elements are possibly present.

In a preferred embodiment of the present invention, the term 'comprising' as used herein is also understood to mean 'consisting of' thereby excluding the presence of other elements besides the explicitly mentioned element.

In a furthermore preferred embodiment, the term 'comprising' as used herein is also understood to mean 'consisting essentially of' thereby excluding the presence of other elements providing a significant contribution to the disclosed teaching besides the explicitly mentioned element.

Further preferred embodiments of the present invention are the subject-matter of the subclaims.

The invention will now be described in some more detail by way of the non-limiting examples and figures.

The sequence protocol shows:
SEQ ID no. 1: wildtype mouse DHFR (dihydrofolate reductase) protein.
SEQ ID no. 2: unspecific temperature-sensitive DHFR protein with all putatively substituted amino acids replaced by Xaa.
SEQ ID no. 3: specific temperature-sensitive DHFR protein with two amino acid substitutions (in the following also termed 'K2').
SEQ ID nos. 4 to 29: primers used for DNA construct design.
SEQ ID no. 30: a linker.
SEQ ID nos. 31 and 32: hemagglutinin epitope.

The figures show:

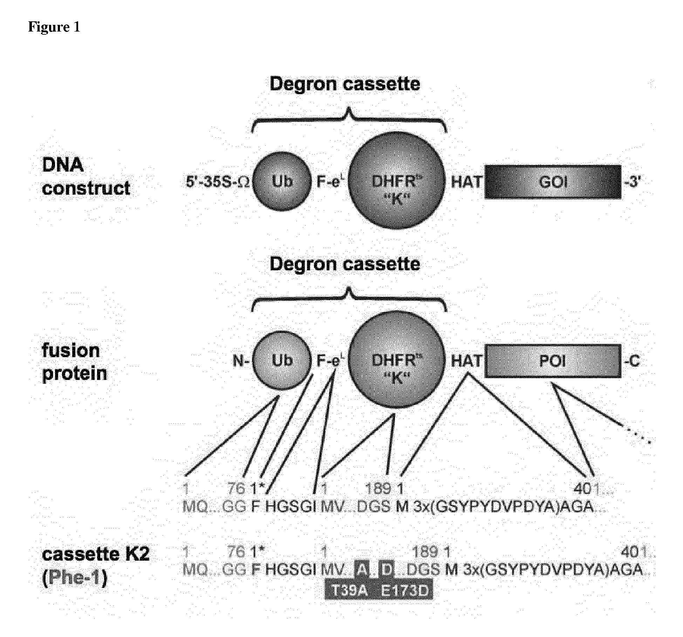

Figure 1: Low-temperature (It) degradation cassette architecture as DNA construct and fusion protein (cassette K2 shows the amino acid substitutions of the temperature-sensitive DHFR protein; Phe-1 is the destabilizing amino acid)

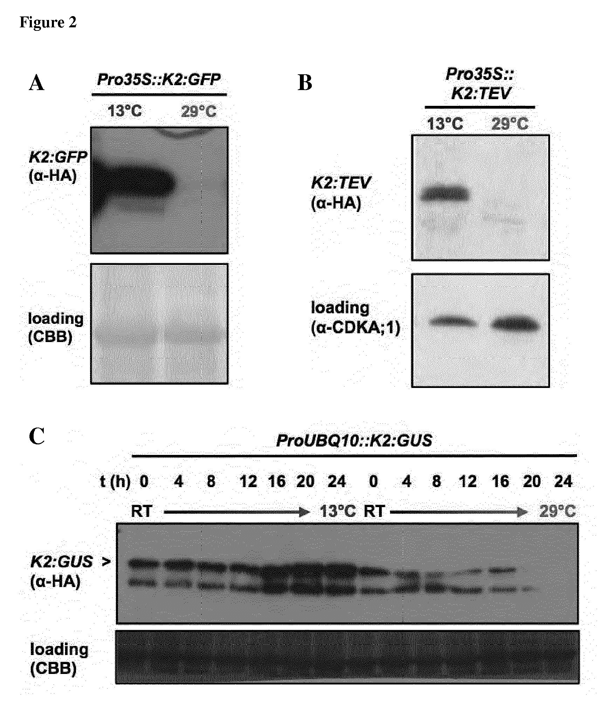

Figure 2: Conditional accumulation and degradation of It-degron fusions in stably transformed *Arabidopsis thaliana* plants grown at permissive or restrictive temperature: (A) K2:GFP (B) K2:TEV and (C) time course of K2:GUS seedlings constitutively grown at ambient temperature (RT) and then shifted to permissive or restrictive temperature. Western blots of material harvested from seedlings grown at permissive and restrictive temperatures. Equal loading was further confirmed by staining of blotted membranes with Coomassie Brilliant Blue G225 after immunostaining or α-PSTAIRE antibody detecting CYCLIN-DEPENDENT KINASE A;1 (CDKA;1).

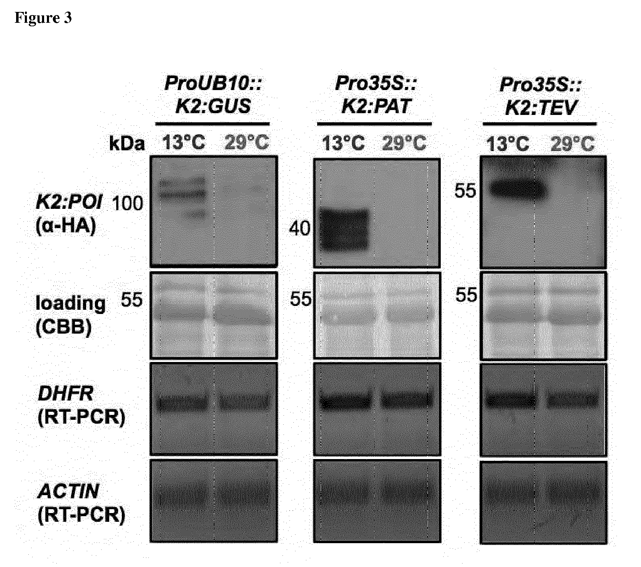

Figure 3: Stability and transcript levels of K2:GUS, K2:PAT, and K2:TEV in transiently transformed *Nicotiana benthamiana* (tobacco) plants.

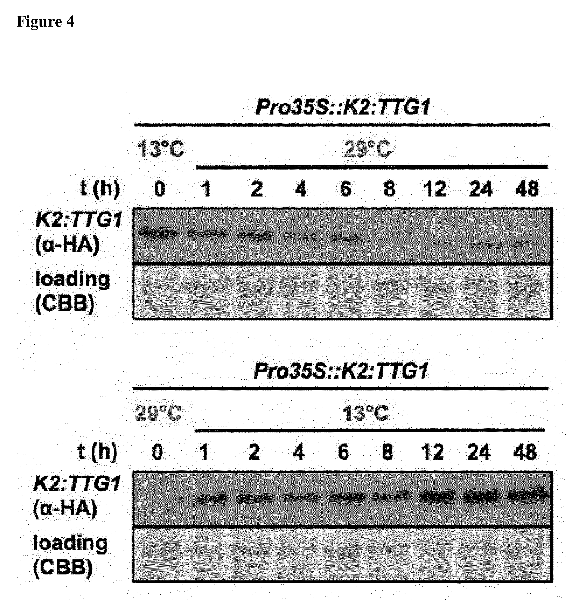

Figure 4: In vivo protein depletion and accumulation time-course of K2:TTG1 from transgenic *ttg1* mutant *Arabidopsis thaliana* plants.

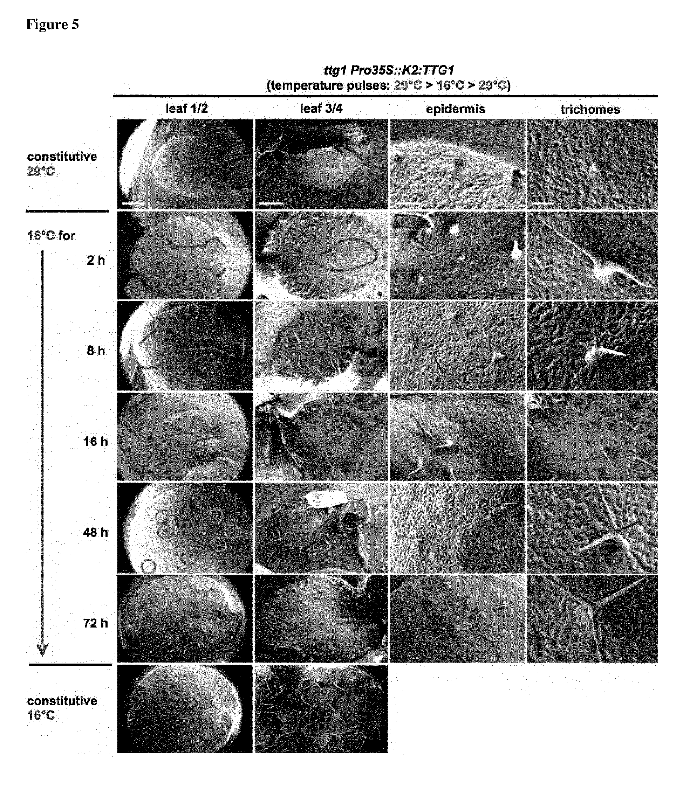

Figure 5: Conditional complementation of *ttg1* with K2:TTG1 depending on the temperature. SEMs of four-week old *ttg1* K2:TTG1 plants. Temperature-pulsing of K2:TTG1 protein in *ttg1* by short shifts from the restrictive to the permissive temperature (16°C). Control plants were grown constitutively at restrictive (29°C, upper row) or permissive (16°C, bottom row) temperatures. Center: plants grown at restrictive and shifted to permissive temperature for the time indicated (2 to 48 h). Red lines: borders of trichome initiation zone moving from lateral to medial with duration of the K2:TTG1 pulse. Green circles: fully mature trichomes and epidermal patterning appearing after 48 h. Arrow heads: loped circumference at trichome bases. Scale bars, 1 mm (left), 500 µm (second and third column), 100 µm (right).

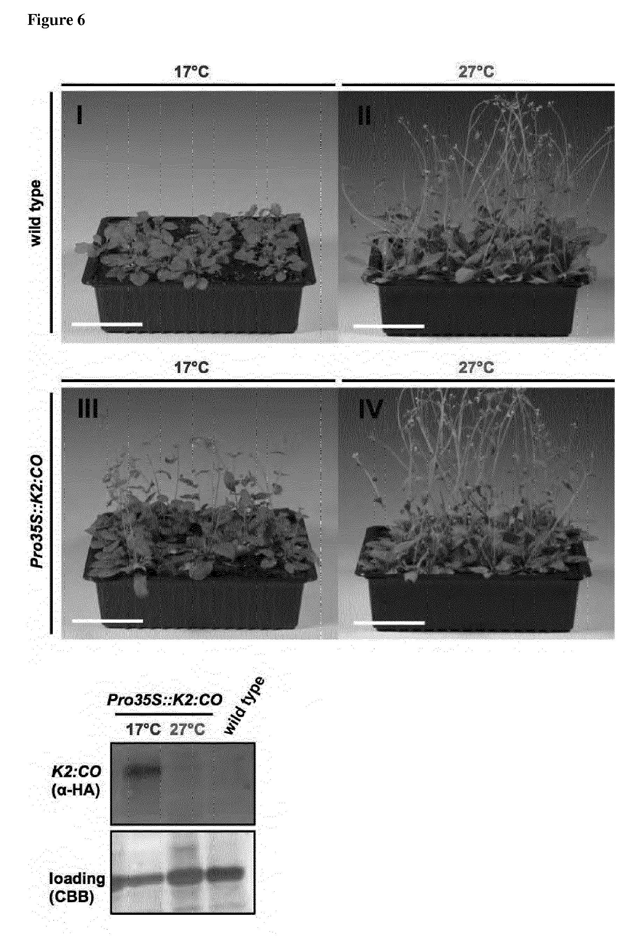

Figure 6: Conditional overexpression of K2:CO fusion leads to premature flowering in vivo. Four-week-old (I), (II) wild type and (III), (IV) K2:CO transgenic plants grown under permissive and restrictive temperatures and short-day conditions. (III) In transgenic K2:CO plants, plants bolted earlier than in the wild type (I). Scale bars, 5 cm. Below: K2:CO protein levels from transgenic plants grown at permissive or restrictive temperatures.

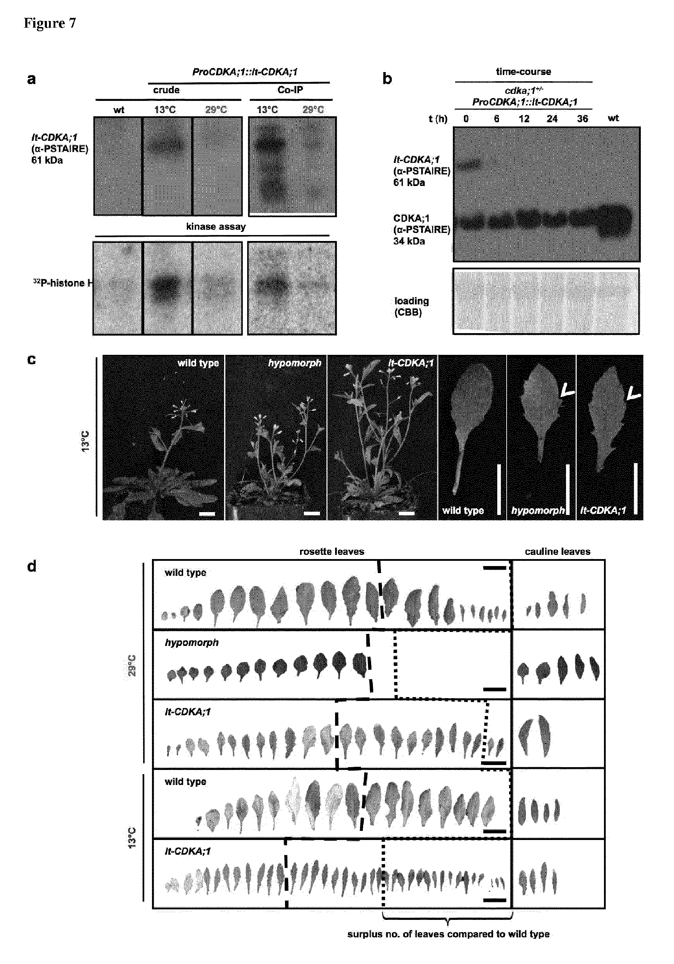

Figure 7: Conditional overexpression of cyclin-dependent kinase CDKA;1. ProCDKA;1::K2-CDKA;1 conditionally complements for a cdka;1 null mutant. (a) Analysis by Western blot and in vitro kinase assay of It-CDKA;1 of stably transformed transgenic plants. (b) Time-course of It-CDKA;1 degradation from heterozygous cdka;1^{+/-} ProCDKA;1::K2:CDKA;1, shifted from permissive (13°C) to restrictive temperature (29°C). Lower bands: endogenous CDKA;1 as housekeeping protein control. (c) Seven-week-old plants grown under permissive conditions (13°C). Left: wild type, hypomorphic CDKA;1T161D allele, K2-CDKA;1. Right: detached rosette leaves. (d) Heteroblastic leaf series of 12-week-old plants: wild type, hypomorph, K2-CDKA;1. Dashed line: threshold of max. no. of rosette leaves in wild type vs. hypomorph; dotted line: max. no. of leaves in wild type vs. It-CDKA;1. Note the additional no. of leaves under permissive conditions in It-CDKA;1 possibly indicative for increased cell division competency due to higher kinase activity.

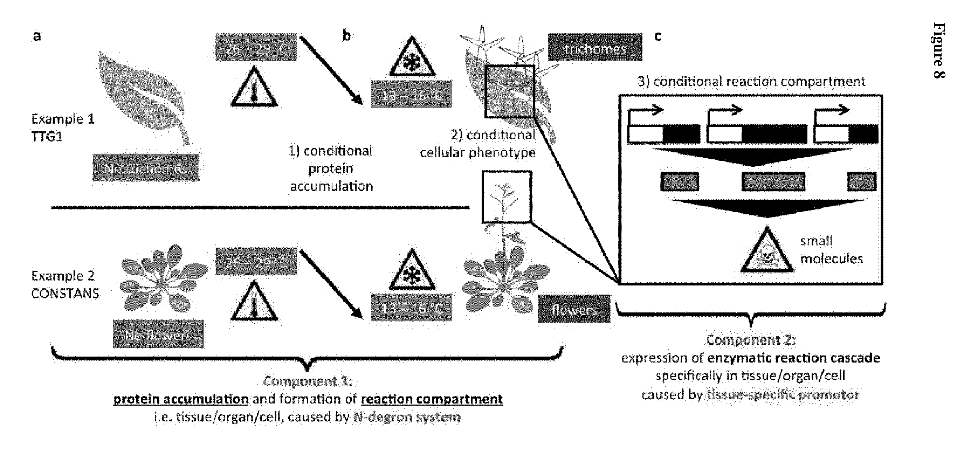

Figure 8: Generation of a conditional reaction compartment which can be used as a conditional bioreactor. (a) Two examples for protein of interest B which both can be conditionally accumulated in vivo and are important molecular factors for organ and tissue specific development are TRANSPARENT TESTA GLABRA1 (TTG1) and CONSTANS (CO). When fused to the It-degron, they are degraded at restrictive temperature but accumulate in vivo at permissive temperature (b). Under these inducing conditions, they fulfill their biological function and trigger the development of specific cell or tissue types, i.e. leaf hairs (trichomes; TTG1) and meristematic tissue, here, apical meristems and flowers (CO). Proteins of interest B fused to the present It-degron thus represent component 1 of the conditional reaction compartment (CRC). (c) The CRC can be used as a production system or bioreactor, e. g. for the synthesis of small molecules and other molecules of interest (MOIs). This component 2 introduces e.g. a reaction cascade via a protein of interest C e.g. as a second transgene or T-DNA containing multiple open reading frames (ORFs). Their expression is restricted to the cell or tissue types which were made conditional by component 1 by using specific promotors such as for trichomes (ProTRIPTYCHON, ProGLABRA2 or ProCAPRICE) or meristematic tissue (ProCDKA;1 or ProWUSCHEL). This combination results in a localized expression of the reaction cascade, i.e. protein of interest C, due to the conditional emergence or absence of the CRC itself. In the figure, three independent ORFs are depicted serving merely as an example where each of them represents a required protein factor or enzyme within the "pipeline" of the reaction cascade. Thus, a CRC can serve as biosynthetic factory, a "bioreactor", within a metabolically highly active biological containment in the context of metabolic engineering and molecular farming.

### Example 1: Molecular modelling of temperature-sensitive DHFR variant DHFR T39A/E173D (K2)

To find the underlying molecular cause for the enhanced temperature-sensitivity of the *K2-DHFR T39A*/*E173D* (SEQ ID No. 3), compared to the classical yeast *DHFR P67L* (K1) and a DHFR protein comprising all three substitutions (K3), we predicted and analysed the impact of the three relevant substitutions by molecular dynamics (MD) and constructed models based on the crystal structure. By calculating root mean standard deviation (RMSD), we found enhanced molecular flexibility in the protein structure of all three substitutions indicating that the mutations lead to increased thermolability and cause a higher intramolecular flexibility between the neighbouring amino acid residues and even within entire domains of the degron-DHFR. MD simulations revealed that none of the three tested DHFR variants undergoes unfolding as suggested previously. To further elucidate the molecular origin of increased thermolability in the variants K2 and K3, we modelled the three different point mutations used in the DHFR variants onto the wildtype structure of DHFR (PDB ID: 1U70). By investigating side chain conformations, we found that T39A does not essentially alter the structure of DHFR in the close vicinity of the point of mutation. Nevertheless, the MD simulations show that Lys69 becomes more flexible and possibly also accessible for ubiquitination. Testing the effect of E173D mutation resulted in a higher flexibility and accessibility of Lys174 which was surprisingly accompanied with conformational changes of the side chains of Arg29 and Lys33.

### Example 2: Temperature-dependent expression of different proteins of interest A

To assess the application spectrum of the It-degron (low-temperature degradation cassette) of the present invention comprising the K2 variant of the DHFR protein (SEQ ID No. 3) in plants, we chose green fluorescent protein (GFP), β-glucuronidase (GUS), and tobacco etch virus protease (TEV) as additional target proteins to be tested when stably transformed in *Arabidopsis. K2:GFP* robustly followed the accumulation/depletion regime **(****Fig. 2****)** and both processes were tracked in time-course experiments with K2 cassettes initiated by either Arg or Phe. Both destabilizing N-termini gave comparable, strongly temperature-dependent results.

Histological stainings for *K2: GUS* revealed activity of the fusion protein *in vivo* at permissive and a significantly reduced activity at restrictive temperature. *K2: GUS* protein levels and activity were strongly decreased for individuals grown constitutively at restrictive temperature with transcript levels unchanged. In time-course experiments, *K2: GUS* protein levels increased and correlated with high enzyme activity after shift to permissive temperature and vice versa if shifted to restrictive conditions. We also identified the *K2: GUS* population with a temperature-dependent hydrolase activity which accumulates at permissive temperature by mass spectrometry. The active *K2: GUS* population accumulated at the restrictive temperature after the addition of proteasome inhibitor to the plants indicating the degradation of the fusion protein via the ubiquitin/proteasome system.

Next, we applied our low temperature approach to TEV as a target, which is widely used for cleaving proteins *in vitro* and *in vivo.* The protease can be expressed for site-specific protein cleavage in many different host organisms without causing adverse effects. In *Arabidopsis, K2:TEV* transcript levels were unaffected by temperature shifts but the fusion protein accumulated exclusively at permissive temperature.

To test the activity *in vivo,* we designed the artificial test substrate *HA:GST-tev-GFP:His* containing epitope-tagged glutathione *S*-transferase and GFP flanking an internal TEV recognition site and transformed *Nicotiana benthamiana* (tobacco) plants with both *K2: TEVand HA:GST-tev-GFP:His.* Here, *K2:TEV* was depleted from plants shifted to 29°C and accumulated in individuals grown at 13°C. Consequently, *K2: TEV* conditionally cleaved the test substrate at permissive temperature. Therefore, a conditional TEV offers opportunities for downstreaming of recombinant proteins by intracellular cleavage. In addition to *K2:TEV,* also *K2: GUS* and a fusion of *K2* with the agriculturally important herbicide resistance phosphinothricin-N-acetyltransferase (PAT) were also expressed in a conditional manner in tobacco **(****Fig. 3****).** Together these findings established that the It-degron approach was suitable also for the manipulation of tobacco and therefore is likely applicable for many more plant species.

### Example 3: Temperature-dependent formation of Arabidopsis thaliana trichomes via expression of a protein of interest B

To assess functionality of the N-terminal degradation cassette in multicellular organisms, we chose easily scorable, quantitative, and irreversible biological readouts after temperature shifts *in vivo,* like the development of *Arabidopsis thaliana* trichomes (leaf hairs) and flower induction (see Example 4). The present temperature-sensitive DHFR (K2) was initiated by phenylalanine, a strongly destabilizing residue in plants. As the first test system, we used the formation of *Arabidopsis* trichomes, which are a well-established model in cell and developmental biology, and an attractive target for plant metabolic engineering. WD40 protein *TRANSPARENT TESTA GLABRA 1 (TTG1)* is an essential regulator for trichome development, *ttg1* mutants are devoid of trichomes but the mutant phenotype can be rescued by expressing *TTG1* under control of the constitutive Cauliflower Mosaic Virus 35S promoter (*Pro35S*). In this evaluation system, the readout for a functional N-terminal degradation cassette is the number of trichomes formed per leaf after the shift from permissive to restrictive temperature. Under permissive conditions, the degradation cassette is non-functional, i.e. the POI (protein of interest) will accumulate and form trichomes, whereas under restrictive conditions, the degradation cassette is active and leads to the degradation of the fusion protein, and leaves will be devoid of trichomes.

Thus, we fused TTG1 to *DHFR T39A*/*E173D* ("K2"), which contains two destabilizing point mutations *T39A*/*E173D.* The expression of *K2:TTG1* caused a conditional, i.e. a temperature-dependent restoration of trichomes in the *ttg1* mutant background. In time-course experiments, decreasing and increasing levels of *K2:TTG1* protein could be tracked as a consequence of temperature up- or down-shifts causing depletion versus accumulation **(****Fig. 4****).** Neither TTG1 protein nor its transcript levels responded to the temperature shifts. Stability predictions, molecular dynamics simulations and modelling of the point mutations within the DHFR crystal structure (see Example 1) indicated that *E173D* contributes a higher conformational flexibility and increases the exposure of Lys side chains on the surface under elevated temperatures. This may lead to a better accessibility to the ubiquitination machinery. Indeed, protein accumulation by this novel low-temperature degradation cassette containing the temperature-sensitive DHFR protein of the present invention was even tuneable and allowed fading out the POI in order to retain minimal doses or to maintain various levels of POI at semi-restrictive temperatures.

The high degree of tunability of *K2:TTG1* and the conditional induction of trichomes in the mutant background allowed us to perform previously impossible experiments, i.e. to dissect the temporal requirement of TTG1 function during trichome establishment and maturation in great detail **(****Fig. 5****).** Taking the pattern of wildtype trichome distribution into account, we found that a pulse of *K2:TTG1* protein is needed for at least two hours to establish a trichome fate during the initial induction phase **(****Fig. 5****).** After a pulse of two hours at a permissive temperature of 16°C, the front of the trichome initiation sites started to move proximal to the midvein and trichomes began to differentiate (branching). Notably, initiation sites develop proximal to the mid-vein as spots with enlarged precursor cells, suggesting that different leaf areas have a differential predisposition to form trichomes. After eight hours, further branch points are formed and few three-branched, albeit not fully developed, trichomes appeared. Many trichomes formed a loped circumference which is typical for de-differentiation if initial maintenance of cell fate is not completed. After 16 hours, a spacing pattern was formed indicating robust trichome maintenance. 48 hours of TTG1 function were needed for developing wild type-like three-branched trichomes. Expansion and partial restoration of a wild type-like distribution pattern as well as morphology of trichomes, is fully accomplished after 72 hours of TTG1 action **(****Fig. 5****).**Here, the present degradation cassette allowed us to determine the temporal requirement of TTG1 in the context of the development of a highly specific cell type. Further, conditional expression of TTG1 in the mutant background allows to establish trichomes as reaction compartments in a temperature dependent manner.

### Example 4: Temperature-dependent initiation of flowering in Arabidopsis thaliana via expression of a protein of interest B

As a second test system with an easily scorable biological readout, we chose wildtype *Arabidopsis* plants that conditionally overexpress the transcription factor CONSTANS (CO), a key regulator of flowering time. Overexpressing CO causes a rapid shift from the vegetative to reproductive growth mode and thereby an early flowering. Thus, at permissive temperatures, plants expressing a functional degron-CO fusion protein *(CO-td)* were expected to flower earlier than plants grown under the restrictive temperature. The readout is hence the days until flowering at permissive temperature in comparison to the wildtype. The It-degron (low-temperature degradation cassette) system very efficiently worked as expression of *K2:CO* at the permissive temperature caused early floral induction. Here, the inducible formation of flower meristems occurred about 14 days earlier than in the wild-type when grown at the same permissive temperature **(****Fig. 6****).** No differences were found when grown at the restrictive temperature as in a time-course experiment, the onset of flowering was observed from day 15 onwards for both wild type and *K2:CO* plants. This phenotype correlates with decreased levels of *K2:CO* under restrictive conditions while transcript levels remained unchanged **(****Fig. 6****).**

Thus, conditionally stable *CO-td,* enabled us to control the onset of flowering and to generate floral meristem cells, i.e. specialized cell types as reaction compartments, on demand.

### Example 5: Temperature-dependent expression of a protein of interest A in Drosophila melanogaster

We then tested the It-degron in *Drosophila melanogaster* using *K2:GFP* and *K2: TEV* as model POIs. *K2:GFP* was destabilized at 16°C in embryonic Kc cells subjected to a temperature shift for 4 h and *K2: TEV* was depleted from macrophage-like Schneider 2 (S2) cells after a shift from 16°C to 29°C. A stable transgenic *Drosophila* line expressing *K2: TEV* under the control of the *Actin5c* promoter proved the principle *in vivo.* After shifting the living flies from the permissive (18°C) to the restrictive (29°C) temperature, we observed that *K2: TEV* becomes instable.

### Materials and methods

### Plants

*Arabidopsis thaliana* (L.) Heynh. plants were sown on soil and grown under standard long-day (16/8 hours light/dark) or short day (8/16 hours light/dark) greenhouse conditions between 18 and 25°C. Seeds were grown aseptically in vitro under long-day regime (16 h light, 8 h dark) on 0.5 % Murashige & Skoog (MS; Duchefa Biochemicals, M0221) containing 1% (w/v) sucrose and 8 g/L phytoagar (Duchefa Biochemicals, P10031). Plants used in this study were all in the background of the Columbia-0 (Col-0; "wildtype" or "wt") accession and either wild-type plants or T-DNA insertion mutants for TTG1 (ttg1, GABI_580A05, NASC stock ID: N455589). For SEMs, 5 to 7 days old plants were treated for the indicated time. First leaves were not yet visible at the time of the pulse experiments. Before protein isolation, transgenic plants were subjected to temperature shifts by removing the potted plants constantly grown at the indicated starting temperature and shifting them to the corresponding destination temperature. To achieve a rapid acclimatization, plants were immediately watered with water prewarmed or precooled to the final temperature.

### DNA work and degron construct design

DNA cloning was performed following standard procedures using Escherichia coli strain DH5α. All fusions were flanked by Gateway attB1 and -2 sites and recombined by BP reactions into pDONR201 (Invitrogen). The three different N-degron cassettes K1 to K3 were based on a 5' synthetic human ubiquitin (Ub) gene (Ecker et al., 1987, J Biol Chem 262: 14213-21) and an Ω leader sequence contained in pRTUB8, a derivative of pRTUB1 (Bachmair et al., 1990, EMBO J 9: 4543-9; Bachmair et al., 1993, Proc Natl Acad Sci USA 90: 418-21). The leader contains the 20 nucleotides upstream of the start codon of tobacco mosaic virus strain U1 (Gallie et al., 1987, Proc Natl Acad Sci U S A 90: 418-21). A triple hemagglutinin tag (HAT) was amplified from pSKTag3SUM6 (kind gift of Andreas Bachmair). Construct K2 is derived from pJH10^{mut} (pJH23), containing a mutated mouse DHFR^{T39A,E173D}, which was isolated in the yeast mutagenesis screen. Ω leader and Ub were amplified with ND70 (ss12attB1UbcoreN - GGGGA CAAGT TTGTA CAAAA AAGCA GGCTT CCTCG AGCTG CAGAA TTACT ATTTA C) and ND78 (as2UbcoreCDHFRovlpN - GATGC AGTTC AATGG TCGAA CCATG ATTCC AGATC CGTGG AACCC ACCTC TAAGT CTTAA GACAA G), DHFR ^{T39A,E173D} from pJH10^{mut} with ND79 (ss2DHFRcoreNUbovlpC - CTTAG AGGTG GGTTC CACGG ATCTG GAATC ATGGT TCGAC CATTG AACTG) and ND80 (as2DHFRovlpCHATcoreN - GTAGG ATCCC ATAGA ACCGTC TTTCT TCTCG T), and HAT with ND81 (ss2HATcoreNovlpDHFRC GAAAG ACGGT TCTAT GGGAT CCTAC CCATA CGAT) and e.g. ND75 (as1234HATcoreCovlpTTG - CTGAA TTATC CATAG CACCA GCACC AGCGT AATCT GGAAC GTCGT ATG) or N138 (asHATNotCO - CTCTC TTGTT TCAAC ATACC AGCGG CCGCA CCAGC GTAAT CTGGA ACGTC GTATG), depending on the fusion partner to follow. For further K2 constructs, an Entry clone containing construct K2:TTG1 was used as a template. A Gateway-compatible Entry clone pEN-L1-K2-L2 containing the K2 degron cassette from Ω leader to shortly after the HAT epitope was generated to construct a K2:POI fusions by Gateway LR reactions. The K2 cassette was amplified from the previously described K2:PAT using primers 21 (K2-Pos2_frw - GCTGC CGCCA TGGGA GGGGA CAAGT TTGTA CAA) and 22 (K2-Pos2_rw-GGGAC CACTT TGTAC AAGAA AGCTG GGTAG GCGCT GCCGC GCGGC A) containing the appropriate attB1/attB2 recombination sites for a Gateway BP reaction.

### Reporter and target proteins in expression constructs

Arabidopsis TTG1 and CO wild-type cDNAs (AT5G24520 and AT5G15840) were amplified with ND76 (ss1234TTGcoreNovlpHATC - GTTCC AGATT ACGCT GGTGC TGGTG CTATG GATAA TTCAG CTCCA GAT) and ND77 (as1234TTGcoreC_attB2 - GGGGA CCACT TTGTA CAAGA AAGCT GGGTC TCAAA CTCTA AGGAG CTGCA T; K2:TTG1, K3:TTG1) and subcloned into pDONR201 (Invitrogen). Entry clones were recombined in an LR reaction into the attR site-containing binary Gateway destination vector pLEELA (Jakoby et al., 2006, Plant Physiol 141: 1293-305), containing a double CaMV 35S promoter or pAM-PAT-GW-ProUBQ10. These backbones carry the bar gene from Streptomyces hygroscopicus that translates to phosphinothricin-N-acetyltransferase (PAT) as plant selection marker conferring resistance towards phosphinotricin (glufosinate ammonium). To generate K2:GFP, the K2 containing Entry clone pEN-L1-K2-L2, see above, was recombined with pAM-Kan-35S-GW-GFP. K2:GUS was assembled by amplifying K2 with ND70 and TR08 (asGUS-HAT - GGGGT TTCTA CAGGA CGTAA CATAG CACCA GCACC AGCGT AATCT GGAAC) from K2:TTG1, as well as by TR07 (ssHAT-GUS - GTTCC AGATT ACGCT GGTGC TGGTG CTATG TTACG TCCTG TAGAA ACCCC) and TR06 (asattB2+GUS - GGGGA CCACT TTGTA CAAGA AAGCT GGGTC TTATT GTTTG CCTCC C) which amplifies GUS from pENTR-gus (uidA CDS in pDONR1). K2:GUS was fused using ND70 and TR06 and introduced into pAM-PAT GW ProUBQ10 by LR reaction. For K2:TEV, K2 was amplified from K2:TTG1 with primers 41 (td-fwd - GGGGA CAAGT TTGTA CAAAA AAGC) and 36 (L4-HArev - CGCTC ATGGG GTGAT GGTGA TGGTG ATGTT TCATA GCGTA ATCTG GAACG TCGTA TG). TEV was isolated from pCT190-6 (Taxis et al., 2009, Mol Syst Biol 5: 267) with primers 46 (LINKER2FORWARD - ATCAC CATCA CCCCA TGAGC GGCCT GGTGC CGCGC GGCAG CGCC) and 29 (TEVREVERSE/TEVrev - TTACC CTTGC GAGTA CACCA ATTCA). By this, a linker sequence ("L4") was generated comprising both triple HA and hexahistidine tags. K2 and TEV were fused with primers 41 and 29. K2:TEV was cloned into pCRII-TOPO (Invitrogen) by TOPO cloning between the XhoI and SpeI sites and then cloned into pAM-PAT-MCS using EcoRI. K2 for K2:PAT was amplified from K2:TTG1 with ND70 and TR11 (asPAT-HAT - GCCGG GCGTC GTTCT GGGCT CATAG CACCA GCACC AGCGT AATCT GGAAC) and PAT from pLEELA with TR10 (ssHAT-PAT - GTTCC AGATT ACGCT GGTGC TGGTG CTATG AGCCC AGAAC GACGC CCGGC) and TR09 (asattB2+PAT - GGGGA CCACT TTGTA CAAGA AAGCT GGGTC TCAGA TTTCG GTGAC GGGCA GGACC GG). The fusion was accomplished with ND70 and TR09. K2:PAT was recombined into pJan33 (double CaMV 35S promoter fused to the first WRYKY33 intron, selectable marker NPTII).

### Drosophila constructs

K2 from pEN-L1-K2-L2 was introduced into pAWG (DGRC; Murphy, T.D., et al. Construction and application of a set of Gateway vectors for expression of tagged proteins in *Drosophila*, unpublished data) via a Gateway LR reaction. ProActin5c::K2:TEV for cell culture transfection and transformation of flies contains DHFR^{T39A,E173D}, amplified from an Entry clone carrying K2:TTG1 using primers 36 and 37 (F-DHFR - TTCCA CGGAT CTGGA ATCAT GG; Phe-K2) or primers 36 and 38 (M-DHFR - ATGCA CGGAT CTGGA ATCAT GG; Met-K2). Instead of the synthetic plant-optimized Ub, a Ub sequence from S. *cerevisiae* (Bachmair et al., 1986, Science 234: 179-86) was used and amplified with primers 9 (Ub-fwd - CACCA TGCAG ATTTT CGTCA AGACT TTGAC) and 39 (F-DHFR-UBrev - CCATG ATTCC AGATC CGTGG AAACC ACCTC TTAGC CTTAG CAC; Phe-K2) or primers 9 and 40 (M-DHFR-UBrev - CCATG ATTCC AGATC CGTGC ATACC ACCTC TTAGC CTTAG CAC; Met-K2). The resulting fragments were fused to Ub:X-DHFR^{T39A,E173D} with primers 9 and 36 (L4-HArev - CGCTC ATGGG GTGAT GGTGA TGGTG ATGTT TCATA GCGTA ATCTG GAACG TCGTA TG). TEV protease was amplified from pCT190-6 with primers 46 (LINKER2FORWARD - ATCAC CATCA CCCCA TGAGC GGCCT GGTGC CGCGC GGCAG CGCC) and 29 (TEVREVERSE/TEVrev-TTACC CTTGC GAGTA CACCA ATTCA) and K2 combined with TEV with primers 9 and 29. The construct was then subcloned in pCRII-TOPO as mentioned above. K2:TEV was prepared as an XhoI-SpeI fragment which was partially cut to leave the internal SpeI site unaffected. The fragment was ligated into pAW (DGRC).

### Plant transformation and selection of transformants

The resulting binary plant Expression vectors were retransformed into Agrobacterium tumefaciens GV3101-pMP90RK (C58C1 Rifr Gmr Kmr) (Koncz and Schell, 1986, Mol Gen Genet 204: 383-96) and transformed by a modified version of the floral dip method (Dissmeyer and Schnittger, 2011, Methods Mol Biol 779: 93-138). For transient transformation of tobacco, leaves of four-week-old plants were infiltrated with Agrobacteria, carrying binary plant expression vectors. Bacteria suspensions were infiltrated into the epidermis on the lower side of the tobacco leaf. To allow for efficient transformation and expression plants were kept for 48 h in the greenhouse, before applying temperature shift experiments by putting them into a growth cabinet at either permissive or restrictive growth conditions. For one data point, 15 leaf discs of 5 mm diameter were harvested from infiltrated areas and snap frozen in liquid nitrogen. Extraction was performed using RIPA buffer as mentioned above for Arabidopsis.

### Drosophila work

*Drosophila melanogaster* Kc (Echalier and Ohanessian, 1969, Methods Mol Biol 779: 93-138) or Schneider 2 (S2) cells (Schneider, 1972J Embryol Exp Morphol 27: 353-65) were grown at 24°C in Schneider's medium (Gibco) supplemented with 10% (v/v) fetal bovine serum (FBS). Cells were passaged into fresh media every 5 days in a 1:10 dilution. Transfection was carried out in a 12-well dish using the Effectene transfection kit (Qiagen) according to the manufacturer's protocol for adherent cells. Temperature shifts were carried out at 15°C or 29°C respectively. Vectors carrying Actin5c::Phe-K2:TEV (F-K2:TEV) or Actin5c::Met-K2:TEV (M-K2:TEV) were injected in ZH-attP-2A or ZH-attP-51D embryos for phiC31 recombinase transgenesis system64 using standard *Drosophila* transgenesis methods. Transformants were grown at 25 °C standard conditions. To analyse constructs activity, adult flies were shifted either at 18 °C (permissive temperature) or 29 °C (restrictive temperature). After 24 h, total protein extracts were obtained from whole flies homogenized in standard lysis buffer.

### Protein extraction and western blot analysis

Tissue of interest (Arabidopsis: leafs or seedlings, tobacco: 20 leaf discs of 5 mm diameter of an infiltrated area) was collected in a standard 2 mL reaction tube containing three Nirosta stainless steel beads (3.175 mm; cat. no. 75306, Mühlmeier) snap frozen in liquid nitrogen and stored at -80°C until use. Material was ground frozen using a bead mill (Retsch; 45 s, 30 Hz) in collection microtube blocks (adaptor set from TissueLyser II, 69984, Qiagen). For K2:TTG1 time-courses, per time-point, one leaf was ground in 200 µL extraction buffer (Tris-Cl 50 mM (pH 7.6), NaCl 150 mM, EDTA 5 mM, SDS 0,1 % (w/v), ß-mercaptoethanol 0,1 % (w/v), EDTA-free Complete Protease Inhibitor Cocktail (Roche Diagnostics)). Alternatively, tissue was lysed using radioimmunoprecipitation assay (RIPA) buffer containing 50 mM Tris-Cl (pH 8), 120 mM NaCl, 20 mM NaF, 1 mM EDTA, 6 mM EGTA, 1 mM benzamidine hydrochloride, 15 mM Na4P2O7, and 1% Nonidet P-40 supplemented with EDTA-free Complete Protease Inhibitor cocktail (Roche Diagnostics) added freshly. Extraction of K2:TEV protease was done in 25 mM Tris-Cl (pH 7.5), 75 mM NaCl, 15 mM MgCl2, 15 mM EGTA pH 8.0, 0.1% (v/v) Tween 20, 0.1% (v/v) Triton X-100, 5 mM DTT and EDTA-free Complete Protease Inhibitor Cocktail (Roche Diagnostics) in a chilled cooling block at 4°C and 800 rpm for 30 min. Insoluble cell debris was pelleted via centrifugation for 20 min at 4°C and 20,000 g, the supernatant was transferred to a fresh tube. Drosophila cells were harvested via centrifugation at 4°C for 5 min at 300 g. The pellet was washed once with ice-cold PBS and cells lysed using RIPA buffer containing 50 mM Tris-Cl pH 8, 120 mM NaCl, 20 mM NaF, 1 mM EDTA, 6 mM EGTA, 1 mM benzamidine hydrochloride 15 mM Na4P2O7, and 1% Nonidet P-40 with EDTA-free Complete Protease Inhibitor cocktail (Roche Diagnostics). Lysis was carried out on a chilled thermo block at 4°C and 800 rpm for 20 min. Insoluble cell debris was pelleted via centrifugation for 20 min at 4°C and 20,000 g, the supernatant was transferred to a fresh tube. S. cerevisiae cultures were grown at 30 °C to logarithmic growth phase, and cycloheximide was added to the cultures at a final concentration of 100 mg/L. Strains carrying temperature-sensitive alleles were expanded at permissive 25 °C and incubated at restrictive 37 °C for 30 min before CHX addition. Yeast proteins were extracted from samples of exponentially growing suspension culture (5-50 mL; OD600 = 0.8-1.2) at the time points indicated by centrifugation and grinding with glass beads at 4°C in 150-300 µL Native lysis buffer (50 mM Na-HEPES (pH 7.5); 150 mM NaCl, 5 mM EDTA, 1 % (v/v) Triton X-100, containing EDTA-free Complete Protease Inhibitor Cocktail (Roche Diagnostics), 20 µM MG132, followed by a second centrifugation (10 min, 4°C, 20,000 g).

### Quantification of glucoronidase activity

Proteins from two-week-old seedlings of K2:GUS plants were extracted in GUS extraction buffer (50 mM Na-phosphate, 10 mM EDTA, 0.1% (w/v) SDS, 0.1% (v/v) Triton X-100, and 10 mM β-mercaptoethanol freshly added) (Kim et al., 2006, Methods Mol Biol 323: 263-73). The GUS extraction buffer was complemented with freshly added EDTA-free protease inhibitor cocktail (Roche). The assay was carried out by mixing 196 µL assay solution containing 1 mM 4-methylumbelliferyl-β-D-glucuronide (4-MUG) with 4 µL of GUS protein extract in a white 96-well plate. Samples were measured for a total of 60 min with one data point taken every minute and normalized to a 4-MU standard. Activity was normalized and determined as pmol 4-MU per min and mg total protein.

### RNA work and RT-PCR

About 50 mg of plant material were used for RNA extraction using the RNeasy Plant Mini Kit (Qiagen). For first-strand cDNA synthesis, 500 ng of total RNA were used with an equimolar mixture of four oligo(dT) primers (CDSIII-NotIA, CDSIII-NotIC, CDSIII-NotIG, CDSIII-NotIT) containing 30 desoxythymidines and XbaI and NotI sites each and RevertAid H Minus Reverse Transcriptase (Thermo Scientific). 1 µL of cDNA was used for subsequent PCR analysis using self-made Taq DNA polymerase. For each sample, two reactions were carried out. One with generic degron specific primers (DHFR_frw and DHFR_rev) to test transcript levels of the transgene.

### Microscopical work

Scanning electron microscopy was done with a SUPRA 40VP (Carl Zeiss MicroImaging) equipped with a K1250X Cryogenic SEM Preparation System (EMITECH), a CPD 030 critical point dryer (Bal-Tec), and SC 7600 sputter coater (Polaron) at the on-campus microscopy core facility Zentrale Mikroskopie (CeMic) of the Max Planck Institute for Plant Breeding Research at Cologne. Light and confocal laser scanning microscopy (CLSM) was performed with an LSM710 system (Carl Zeiss MicroImaging). K2:GFP fluorescence was observed in root tips of plants that were aseptically grown for 2 weeks under long-day conditions at either constitutively restrictive (13°C) or permissive (29°C) temperatures. Photographs of in vitro cultures and histological stainings were taken with a stereo microscope (Stemi 2000-C, Carl Zeiss MicroImaging) equipped with a Zeiss CL 6000 LED illumination unit, and a video adapter 60 C including an AxioCam ERc 5s digital camera (Carl Zeiss MicroImaging).

## Claims

1. A process for producing a temperature-sensitive conditional mutant of a multicellular organism comprising the steps of:
a) providing a low-temperature N-terminal degradation cassette or a vector comprising it and at least one cell or part of a multicellular organism,
b) transfecting or transforming the at least one cell or part of the multicellular organism with the low-temperature N-terminal degradation cassette or vector, and
c) obtaining a temperature-sensitive conditional mutant of the multicellular organism or part thereof.

2. The process according to claim 1, wherein the degradation cassette comprises:
i) a nucleotide sequence encoding a temperature-sensitive dihydrofolate reductase (DHFR) protein, which is a DHFR protein, in which at least one amino acid selected from the group consisting of the residues corresponding to threonine 39, valine 51, isoleucine 52, methionine 53, valine 75, valine 113, tryptophan 114, isoleucine 115, leucine 134, phenylalanine 135, valine 136, isoleucine 139 and glutamic acid 173 of the wildtype mouse DHFR protein according to SEQ ID No. 1 is substituted,
ii) a codon for a destabilizing amino acid selected from the group consisting of arginine, lysine, histidine, phenylalanine, tyrosine, tryptophan, leucine, isoleucine, methionine, aspartic acid, glutamic acid, asparagine, glutamine and cysteine which codon is located 5' of the nucleotide sequence encoding the temperature-sensitive DHFR protein identified in i), and
iii) a nucleotide sequence encoding a ubiquitin moiety which is located 5' of the codon for the destabilizing amino acid identified in ii).

3. The process according to claim 1 or 2, wherein the degradation cassette comprises:
iv) a nucleotide sequence encoding a protein of interest A, B or a combination thereof which is located 3' of the nucleotide sequence encoding the temperature-sensitive DHFR protein identified in i).

4. The process according to any one of the preceding claims, wherein the protein of interest A, B or the combination thereof is degradable upon shifting the temperature from a permissive lower temperature to a restrictive higher temperature for a period of time sufficient to facilitate degradation of the protein of interest A, B or the combination thereof.

5. The process according to claim 3 or 4, wherein the protein of interest B is able to control the development of a reaction compartment and wherein in step c) a multicellular organism is obtained which is able to develop the reaction compartment temperature-dependently.

6. The process according to claim 5, wherein the reaction compartment is a specific tissue or cell type of the multicellular organism.

7. The process according to any one of the preceding claims, wherein the at least one cell or part of the multicellular organism provided in step a) lacks a functional endogenous gene encoding the protein of interest A or B.

8. The process according to any one of claims 5 to 7, wherein the at least one cell or part of the multicellular organism is transfected or transformed in step b) with a degradation cassette comprising a nucleotide sequence encoding at least the protein of interest B and simultaneously or in a further step d) with a vector comprising a nucleotide sequence encoding at least a protein of interest C which nucleotide sequence is under the control of a promotor specific for the reaction compartment and wherein in step c) or a further step e) a multicellular organism is obtained which is able to develop the reaction compartment and to express the protein of interest C temperature-dependently.

9. The process according to claim 8, wherein the protein of interest C is part of an enzymatic cascade or at least one regulatory element thereof able to generate a molecule of interest.

10. The process according to any one of the preceding claims, wherein the permissive temperature is below 24 °C and the restrictive temperature is at least 24 °C.

11. The process according to any one of the preceding claims, wherein the multicellular organism is a plant or an insect.

12. The process according to any one of claims 2 to 11, wherein the DHFR protein is the wildtype mouse DHFR protein and the temperature-sensitive DHFR protein is represented by SEQ ID No. 2.

13. The process according to any one of claims 2 to 12, wherein in case any of the valine residues 51, 75, 113 and 136 is substituted, then it is substituted with an amino acid selected from the group consisting of glycine, asparagine and threonine; in case any of the residues isoleucine 52, methionine 53, tryptophan 114, isoleucine 115, leucine 134, phenylalanine 135 and isoleucine 139 is substituted, then it is substituted with an amino acid selected from the group consisting of glycine, glutamic acid and asparagine; in case the threonine residue 39 is substituted, then it is substituted with alanine and in case the glutamic acid residue 173 is substituted, then it is substituted with aspartic acid.

14. The process according to any one of claims 2 to 13, wherein the threonine residue 39 is substituted with alanine and/or the glutamic acid residue 173 is substituted with aspartic acid, preferably both amino acids are substituted as represented by SEQ ID No. 3.

15. The process according to any one of claims 2 to 14, wherein the degradation cassette comprises:
v) a nucleotide sequence encoding a linker comprising the amino acids histidine, glycine, serine, glycine and isoleucine which nucleotide sequence is located 5' of the nucleotide sequence encoding the temperature-sensitive DHFR protein identified in i) and 3' of the codon for the destabilizing amino acid identified in ii).

16. The process according to any one of claims 2 to 15, wherein the degradation cassette comprises:
vi) a nucleotide sequence encoding a triple hemagglutinin epitope which nucleotide sequence is located 3' of the nucleotide sequence encoding the temperature-sensitive DHFR protein identified in i) and 5' of the nucleotide sequence encoding the protein of interest A, B or the combination thereof identified in iv).

17. A low-temperature N-terminal degradation cassette comprising:
i) a nucleotide sequence encoding a temperature-sensitive DHFR protein, which is a DHFR protein, in which at least one amino acid selected from the group consisting of the residues corresponding to threonine 39, valine 51, isoleucine 52, methionine 53, valine 75, valine 113, tryptophan 114, isoleucine 115, leucine 134, phenylalanine 135, valine 136, isoleucine 139 and glutamic acid 173 of the wildtype mouse DHFR protein according to SEQ ID No. 1 is substituted,
ii) a codon for a destabilizing amino acid selected from the group consisting of arginine, lysine, histidine, phenylalanine, tyrosine, tryptophan, leucine, isoleucine, methionine, aspartic acid, glutamic acid, asparagine, glutamine and cysteine which codon is located 5' of the nucleotide sequence encoding the temperature-sensitive DHFR protein identified in i), and
iii) a nucleotide sequence encoding a ubiquitin moiety which is located 5' of the codon for the destabilizing amino acid identified in ii).

18. The degradation cassette according to claim 17, wherein the DHFR protein is the wildtype mouse DHFR protein and the temperature-sensitive DHFR protein is represented by SEQ ID No. 2.

19. The degradation cassette according to claim 17 or 18, wherein in case any of the valine residues 51, 75, 113 and 136 is substituted, then it is substituted with an amino acid selected from the group consisting of glycine, asparagine and threonine; in case any of the residues isoleucine 52, methionine 53, tryptophan 114, isoleucine 115, leucine 134, phenylalanine 135 and isoleucine 139 is substituted, then it is substituted with an amino acid selected from the group consisting of glycine, glutamic acid and asparagine; in case the threonine residue 39 is substituted, then it is substituted with alanine and in case the glutamic acid residue 173 is substituted, then it is substituted with aspartic acid.

20. The degradation cassette according to any one of claims 17 to 19, wherein the threonine residue 39 is substituted with alanine and/or the glutamic acid residue 173 is substituted with aspartic acid, preferably both amino acids are substituted as represented by SEQ ID No. 3.

21. The degradation cassette according to any one of claims 17 to 20, wherein the degradation cassette comprises:
iv) a nucleotide sequence encoding the protein of interest A, B or a combination thereof which is located 3' of the nucleotide sequence encoding the temperature-sensitive DHFR protein identified in i).

22. The degradation cassette according to any one of claims 17 to 21, wherein the degradation cassette comprises:
v) a nucleotide sequence encoding a linker comprising the amino acids histidine, glycine, serine, glycine and isoleucine which nucleotide sequence is located 5' of the nucleotide sequence encoding the temperature-sensitive DHFR protein identified in i) and 3' of the codon for the destabilizing amino acid identified in ii).

23. The degradation cassette according to any one of claims 17 to 22, wherein the degradation cassette comprises:
vi) a nucleotide sequence encoding a triple hemagglutinin epitope which nucleotide sequence is located 3' of the nucleotide sequence encoding the temperature-sensitive DHFR protein identified in i) and 5' of the nucleotide sequence encoding the protein of interest A, B or the combination thereof identified in iv).


SEQUENCE LISTING

<110>  Leibniz-Institut für Pflanzenbiochemie und Institute de
       Biologie Moléculaire des Plantes

<120>  Rapid depletion and reversible accumulation of proteins in vivo

<130>  204767

<160>  32

<170>  PatentIn version 3.5

<210>  1
<211>  187
<212>  PRT
<213>  mus musculus

<400>  1

Met Val Arg Pro Leu Asn Cys Ile Val Ala Val Ser Gln Asn Met Gly
1               5                   10                  15

Ile Gly Lys Asn Gly Asp Leu Pro Trp Pro Pro Leu Arg Asn Glu Phe
            20                  25                  30

Lys Tyr Phe Gln Arg Met Thr Thr Thr Ser Ser Val Glu Gly Lys Gln
        35                  40                  45

Asn Leu Val Ile Met Gly Arg Lys Thr Trp Phe Ser Ile Pro Glu Lys
    50                  55                  60

Asn Arg Pro Leu Lys Asp Arg Ile Asn Ile Val Leu Ser Arg Glu Leu
65                  70                  75                  80

Lys Glu Pro Pro Arg Gly Ala His Phe Leu Ala Lys Ser Leu Asp Asp
                85                  90                  95

Ala Leu Arg Leu Ile Glu Gln Pro Glu Leu Ala Ser Lys Val Asp Met
            100                 105                 110

Val Trp Ile Val Gly Gly Ser Ser Val Tyr Gln Glu Ala Met Asn Gln
        115                 120                 125

Pro Gly His Leu Arg Leu Phe Val Thr Arg Ile Met Gln Glu Phe Glu
    130                 135                 140

Ser Asp Thr Phe Phe Pro Glu Ile Asp Leu Gly Lys Tyr Lys Leu Leu
145                 150                 155                 160

Pro Glu Tyr Pro Gly Val Leu Ser Glu Val Gln Glu Glu Lys Gly Ile
                165                 170                 175

Lys Tyr Lys Phe Glu Val Tyr Glu Lys Lys Asp
            180                 185

<210>  2
<211>  187
<212>  PRT
<213>  mus musculus

<220>
<221>  misc_feature
<222>  (39)..(39)
<223>  Xaa can be any naturally occurring amino acid

<220>
<221>  misc_feature
<222>  (51)..(53)
<223>  Xaa can be any naturally occurring amino acid

<220>
<221>  misc_feature
<222>  (75)..(75)
<223>  Xaa can be any naturally occurring amino acid

<220>
<221>  misc_feature
<222>  (113)..(115)
<223>  Xaa can be any naturally occurring amino acid

<220>
<221>  misc_feature
<222>  (134)..(136)
<223>  Xaa can be any naturally occurring amino acid

<220>
<221>  misc_feature
<222>  (139)..(139)
<223>  Xaa can be any naturally occurring amino acid

<220>
<221>  misc_feature
<222>  (173)..(173)
<223>  Xaa can be any naturally occurring amino acid

<400>  2

Met Val Arg Pro Leu Asn Cys Ile Val Ala Val Ser Gln Asn Met Gly
1               5                   10                  15

Ile Gly Lys Asn Gly Asp Leu Pro Trp Pro Pro Leu Arg Asn Glu Phe
            20                  25                  30

Lys Tyr Phe Gln Arg Met Xaa Thr Thr Ser Ser Val Glu Gly Lys Gln
        35                  40                  45

Asn Leu Xaa Xaa Xaa Gly Arg Lys Thr Trp Phe Ser Ile Pro Glu Lys
    50                  55                  60

Asn Arg Pro Leu Lys Asp Arg Ile Asn Ile Xaa Leu Ser Arg Glu Leu
65                  70                  75                  80

Lys Glu Pro Pro Arg Gly Ala His Phe Leu Ala Lys Ser Leu Asp Asp
                85                  90                  95

Ala Leu Arg Leu Ile Glu Gln Pro Glu Leu Ala Ser Lys Val Asp Met
            100                 105                 110

Xaa Xaa Xaa Val Gly Gly Ser Ser Val Tyr Gln Glu Ala Met Asn Gln
        115                 120                 125

Pro Gly His Leu Arg Xaa Xaa Xaa Thr Arg Xaa Met Gln Glu Phe Glu
    130                 135                 140

Ser Asp Thr Phe Phe Pro Glu Ile Asp Leu Gly Lys Tyr Lys Leu Leu
145                 150                 155                 160

Pro Glu Tyr Pro Gly Val Leu Ser Glu Val Gln Glu Xaa Lys Gly Ile
                165                 170                 175

Lys Tyr Lys Phe Glu Val Tyr Glu Lys Lys Asp
            180                 185

<210>  3
<211>  187
<212>  PRT
<213>  mus musculus

<400>  3

Met Val Arg Pro Leu Asn Cys Ile Val Ala Val Ser Gln Asn Met Gly
1               5                   10                  15

Ile Gly Lys Asn Gly Asp Leu Pro Trp Pro Pro Leu Arg Asn Glu Phe
            20                  25                  30

Lys Tyr Phe Gln Arg Met Ala Thr Thr Ser Ser Val Glu Gly Lys Gln
        35                  40                  45

Asn Leu Val Ile Met Gly Arg Lys Thr Trp Phe Ser Ile Pro Glu Lys
    50                  55                  60

Asn Arg Pro Leu Lys Asp Arg Ile Asn Ile Val Leu Ser Arg Glu Leu
65                  70                  75                  80

Lys Glu Pro Pro Arg Gly Ala His Phe Leu Ala Lys Ser Leu Asp Asp
                85                  90                  95

Ala Leu Arg Leu Ile Glu Gln Pro Glu Leu Ala Ser Lys Val Asp Met
            100                 105                 110

Val Trp Ile Val Gly Gly Ser Ser Val Tyr Gln Glu Ala Met Asn Gln
        115                 120                 125

Pro Gly His Leu Arg Leu Phe Val Thr Arg Ile Met Gln Glu Phe Glu
    130                 135                 140

Ser Asp Thr Phe Phe Pro Glu Ile Asp Leu Gly Lys Tyr Lys Leu Leu
145                 150                 155                 160

Pro Glu Tyr Pro Gly Val Leu Ser Glu Val Gln Glu Asp Lys Gly Ile
                165                 170                 175

Lys Tyr Lys Phe Glu Val Tyr Glu Lys Lys Asp
            180                 185

<210>  4
<211>  56
<212>  DNA
<213>  artificial sequence

<220>
<223>  ND70

<400>  4
ggggacaagt ttgtacaaaa aagcaggctt cctcgagctg cagaattact atttac          56

<210>  5
<211>  66
<212>  DNA
<213>  artificial sequence

<220>
<223>  ND78

<400>  5
gatgcagttc aatggtcgaa ccatgattcc agatccgtgg aacccacctc taagtcttaa      60

gacaag                                                                  66

<210>  6
<211>  50
<212>  DNA
<213>  artificial sequence

<220>
<223>  ND79

<400>  6
cttagaggtg ggttccacgg atctggaatc atggttcgac cattgaactg                  50

<210>  7
<211>  32
<212>  DNA
<213>  artificial sequence

<220>
<223>  ND80

<400>  7
gtaggatccc atagaaccgt ctttcttctc gt                                    32


<210>  8
<211>  34
<212>  DNA
<213>  artificial sequence

<220>
<223>  ND81

<400>  8
gaaagacggt tctatgggat cctacccata cgat                                  34


<210>  9
<211>  48
<212>  DNA
<213>  artificial sequence

<220>
<223>  ND75

<400>  9
ctgaattatc catagcacca gcaccagcgt aatctggaac gtcgtatg                   48


<210>  10
<211>  55
<212>  DNA
<213>  artificial sequence

<220>
<223>  N138

<400>  10
ctctcttgtt tcaacatacc agcggccgca ccagcgtaat ctggaacgtc gtatg           55


<210>  11
<211>  33
<212>  DNA
<213>  artificial sequence

<220>
<223>  Primer 21

<400>  11
gctgccgcca tgggagggga caagtttgta caa                                   33


<210>  12
<211>  46
<212>  DNA

<213>  artificial sequence

<220>
<223>  Primer 22

<400>  12
gggaccactt tgtacaagaa agctgggtag gcgctgccgc gcggca                    46


<210>  13
<211>  48
<212>  DNA
<213>  artificial sequence

<220>
<223>  ND76

<400>  13
gttccagatt acgctggtgc tggtgctatg gataattcag ctccagat                  48


<210>  14
<211>  51
<212>  DNA
<213>  artificial sequence

<220>
<223>  ND77

<400>  14
ggggaccact ttgtacaaga aagctgggtc tcaaactcta aggagctgca t              51


<210>  15
<211>  50
<212>  DNA
<213>  artificial sequence

<220>
<223>  TR08

<400>  15
ggggtttcta caggacgtaa catagcacca gcaccagcgt aatctggaac                50


<210>  16
<211>  50
<212>  DNA
<213>  artificial sequence

<220>
<223>  TR07

<400>  16
gttccagatt acgctggtgc tggtgctatg ttacgtcctg tagaaacccc                50


<210>  17
<211>  46
<212>  DNA
<213>  artificial sequence

<220>

<223>  TR06

<400>  17
ggggaccact ttgtacaaga aagctgggtc ttattgtttg cctccc                    46


<210>  18
<211>  24
<212>  DNA
<213>  artificial sequence

<220>
<223>  Primer 41

<400>  18
ggggacaagt ttgtacaaaa aagc                                            24


<210>  19
<211>  57
<212>  DNA
<213>  artificial sequence

<220>
<223>  Primer 36

<400>  19
cgctcatggg gtgatggtga tggtgatgtt tcatagcgta atctggaacg tcgtatg       57


<210>  20
<211>  44
<212>  DNA
<213>  artificial sequence

<220>
<223>  Primer 46

<400>  20
atcaccatca ccccatgagc ggcctggtgc cgcgcggcag cgcc                      44


<210>  21
<211>  25
<212>  DNA
<213>  artificial sequence

<220>
<223>  Primer 29

<400>  21
ttacccttgc gagtacacca attca                                           25


<210>  22
<211>  50
<212>  DNA
<213>  artificial sequence

<220>
<223>  TR11

<400>  22

gccgggcgtc gttctgggct catagcacca gcaccagcgt aatctggaac 50

<210> 23
<211> 50
<212> DNA
<213> artificial sequence

<220>
<223> TR10

<400> 23
gttccagatt acgctggtgc tggtgctatg agcccagaac gacgcccggc 50

<210> 24
<211> 57
<212> DNA
<213> artificial sequence

<220>
<223> TR09

<400> 24
ggggaccact ttgtacaaga aagctgggtc tcagatttcg gtgacgggca ggaccgg 57

<210> 25
<211> 22
<212> DNA
<213> artificial sequence

<220>
<223> Primer 37

<400> 25
ttccacggat ctggaatcat gg 22

<210> 26
<211> 22
<212> DNA
<213> artificial sequence

<220>
<223> Primer 38

<400> 26
atgcacggat ctggaatcat gg 22

<210> 27
<211> 30
<212> DNA
<213> artificial sequence

<220>
<223> Primer 9

<400> 27
caccatgcag attttcgtca agactttgac 30

<210>  28
<211>  43
<212>  DNA
<213>  artificial sequence

<220>
<223>  Primer 39

<400>  28
ccatgattcc agatccgtgg aaaccacctc ttagccttag cac                                43


<210>  29
<211>  43
<212>  DNA
<213>  artificial sequence

<220>
<223>  Primer 40

<400>  29
ccatgattcc agatccgtgc ataccacctc ttagccttag cac                                43


<210>  30
<211>  5
<212>  PRT
<213>  artificial sequence

<220>
<223>  Linker

<400>  30

His Gly Ser Gly Ile
1               5


<210>  31
<211>  9
<212>  PRT
<213>  artificial sequence

<220>
<223>  hemagglutinin epitope

<400>  31

Tyr Pro Tyr Asp Val Pro Asp Tyr Ala
1               5


<210>  32
<211>  11
<212>  PRT
<213>  artificial sequence

<220>
<223>  hemagglutinin epitope

<400>  32

Gly Ser Tyr Pro Tyr Asp Val Pro Asp Tyr Ala

1                    5                        10